# EUROPEAN PATENT SPECIFICATION

(11) **EP 4 530 979 B1**
(45) Date of publication and mention of the grant of the patent: **13.05.2026**
(21) Application number: 24202588.0
(22) Date of filing: 25.09.2024
(51) Int. Cl.: G06T 7/00, G16H 30/40

(54) **DATA GENERATION PROGRAM, DATA GENERATION METHOD, AND DATA GENERATION APPARATUS**
DATENERZEUGUNGSPROGRAMM, DATENERZEUGUNGSVERFAHREN UND DATENERZEUGUNGSVORRICHTUNG
PROGRAMME DE GÉNÉRATION DE DONNÉES, PROCÉDÉ DE GÉNÉRATION DE DONNÉES ET APPAREIL DE GÉNÉRATION DE DONNÉES

(30) Priority: 28.09.2023 JP 2023167786
(43) Date of publication of application: 02.04.2025
(73) Proprietor: J. MORITA MFG. CORP., Fushimi-ku Kyoto-shi Kyoto 612-8533 (JP)
(72) Inventor: YOSHIKAWA, Hideki, Kyoto, 612-8533 (JP)
(74) Representative: Müller Hoffmann & Partner

(56) References cited:
- KR-A- 20230 018 874
- US-A1- 2014 234 796
- US-B2- 10 201 318

## Description

### CROSS REFERENCE TO RELATED APPLICATIONS

This application is based on Japanese Patent Application No. 2023-167786 filed with the Japan Patent Office on September 28, 2023.

### BACKGROUND

### Field

The present disclosure relates to a data generation program, a data generation method, and a data generation apparatus configured to generate image data for checking a state of a biological tissue including a tooth and a gingiva.

### Description of the Background Art

A periodontal disease is an infectious inflammatory disease caused by periodontal pathogenic bacteria. The periodontal disease includes diseases caused in the periodontium composed of a gingiva, cementum, a periodontal membrane, and an alveolar bone, necrotizing periodontal diseases, abscesses of the periodontium, combined periodontic-endodontic lesions, gingival recession (for example, lowering of the gingiva), recession of the alveolar bone (for example, lowering of the alveolar bone), and occlusal trauma caused by strong occlusal force or abnormal force. Progress of the periodontal disease causes inflammation of the gingiva, tooth mobility, or recession of the alveolar bone. With progress of recession of the alveolar bone, standing of a tooth becomes unstable and the tooth may come off.

A method of measuring a crown root ratio is used for evaluation of stability of the tooth affected by the periodontal disease. This method uses a ratio between a length of a root portion of a tooth buried in the alveolar bone and a length of a crown portion of the tooth exposed on the outside of the alveolar bone, as an index of evaluation of stability of the tooth. Such a crown root ratio has conventionally usually been measured based on combination of examination of a radiographic image and measurement of a periodontal pocket or the like. For example, Japanese Patent Laying-Open No. 10-174693 discloses examination of a state of a tooth and a gingiva by measurement by an operator such as a dentist, of a depth of a periodontal pocket by insertion of a hand-held probe into the periodontal pocket.
Further periodontal disease measurement methods are known from US 2014/234796 A1.

### SUMMARY

In a method of examination for a periodontal disease disclosed in Japanese Patent Laying-Open No. 10-174693, the probe should be inserted in the periodontal pocket, which imposes great burdens on a patient. In addition, a result of examination for the periodontal disease may vary depending on skills of an operator who measures the depth of the periodontal pocket. The probe is inserted in and taken out of the periodontal pocket where pathogenic bacteria are present. Therefore, when the pathogenic bacteria enter a tooth with minor or no symptom via the probe, the tooth may be infected by the periodontal disease. Furthermore, in measurement of the depth of the periodontal pocket, the patient may feel pain or experience bleeding. When the patient experiences bleeding, the pathogenic bacteria may also enter the blood. When the depth of the periodontal pocket is measured at a plurality of locations of each tooth, a time period for measurement may be long. In measurement of the crown root ratio in the examination of the radiographic image, only a state of the tooth viewed in a direction of incidence of X-rays has been observed and only an image superimposed in the direction of incidence of X-rays has been obtained, and hence it is difficult to accurately measure the crown root ratio.

The present disclosure was made to solve such a problem, and an object thereof is to provide a technique that allows easy and accurate measurement of a crown root ratio.

The above problem is solved by the claimed subject-matter which defines the present invention. Embodiments not covered by the claimed subject-matter do not form part of the invention.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a diagram showing an application of a data generation apparatus.
Fig. 2 is a block diagram showing a configuration of the data generation apparatus of Fig. 1.
Fig. 3 is a diagram for illustrating setting of a measurement direction and each plane for measurement of a crown root ratio.
Figs. 4 to 6 are each a diagram for illustrating exemplary setting of the crown root ratio.
Fig. 7 is a diagram for illustrating an exemplary reference surface set in an oral cavity image.
Fig. 8 is a flowchart for illustrating exemplary data generation processing performed by the data generation apparatus of Fig. 1.
Fig. 9A is a diagram showing exemplary use of a bounding box.
Fig. 9B is a diagram showing exemplary use of a bounding box.
Fig. 9C is a diagram showing exemplary use of a bounding box.

### DESCRIPTION

In the below description the same or corresponding elements in the drawings have the same reference characters allotted and description thereof will not be repeated.

### [Application]

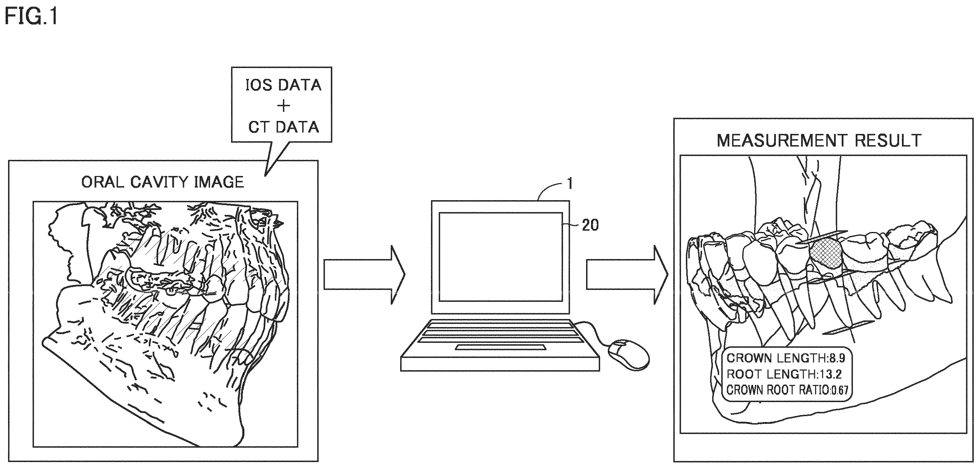

An application of a data generation apparatus 1 will be described with reference to Fig. 1. Fig. 1 is a diagram showing an application of data generation apparatus 1. In a preparation stage, a user of data generation apparatus 1 obtains three-dimensional data (optical scanner data) including position information of each point in a point group (a plurality of points) indicating a surface of a biological tissue including a tooth and a gingiva in an oral cavity by scanning the inside of the oral cavity of a subject with the use of a not-shown three-dimensional scanner (optical scanner). The three-dimensional data includes as position information, a coordinate (X, Y, Z) of each point indicating the surface of the biological tissue in an X-axis direction, a Y-axis direction, and a Z-axis direction that are predetermined. Directions in the position information described above may be, for example, such that the X-axis direction is a lateral direction, the Y-axis direction is a longitudinal direction, and the Z-axis direction is a height direction. Furthermore, the three-dimensional data may include color information indicating an actual color of a portion (a surface portion of the biological tissue) corresponding to each point in the point group (the plurality of points) indicating the surface of the biological tissue including the tooth and the gingiva in the oral cavity.

The "user" includes an operator (a doctor or the like) or an assistant (a dental nurse, a dental technician, a nurse, or the like) in various fields such as dentistry, dental surgery, orthopedics, plastic surgery, and cosmetic surgery. The "subject" includes a patient of dentistry, dental surgery, orthopedics, plastic surgery, and cosmetic surgery. An expression "a user does something" below encompasses an example in which a user gives an execution command to have an apparatus or a program perform processing or operate.

The three-dimensional scanner is what is called an intraoral scanner capable of optically picking up an image of the inside of the oral cavity of a subject by a confocal method or triangulation, and it is capable of obtaining position information of each point in the point group that defines the surface of the biological tissue (for example, the tooth and the gingiva in the oral cavity) set in a certain coordinate space that is to be scanned. Optical imaging and scanning of the inside of the oral cavity is also referred to as intraoral scan (IOS). The user can generate a rendered image (an appearance image) showing a three-dimensional geometry of the biological tissue based on three-dimensional data obtained by the three-dimensional scanner. The "rendered image" is an image generated by processing or edition of certain data. For example, the user can generate a rendered image showing a biological tissue (a part of the biological tissue that can be shown based on IOS data) viewed from a prescribed point of view by processing or edition of three-dimensional data of the biological tissue obtained by the three-dimensional scanner. Furthermore, the user can generate, by varying the prescribed point of view in multiple directions, a plurality of rendered images showing the biological tissue viewed in multiple directions. The rendered image may be a two-dimensional image. The two-dimensional image may be an image obtained by projection of image data on a projection plane for coordinate operation that intersects in a direction of line of sight. For example, the rendered image may be generated as a three-dimensional image that can stereoscopically be observed with a viewer such as general-purpose VR goggles.

The user can also obtain three-dimensional volume (voxel) data of a hard tissue portion (a bone, a tooth, or the like) around a maxilla and a mandible of a subject by imaging the maxilla and the mandible of the subject with a not-shown computed tomography (CT) scanner. The CT scanner is "X-ray equipment" that takes a CT of the maxilla and the mandible of the subject by rotation around the face of the subject, of a transmitter and a receiver of X rays which are a kind of radioactive rays. The user can generate the rendered image (a CT image or an appearance image) showing the three-dimensional geometry of the biological tissue based on volume data obtained by the CT scanner, of the biological tissue which is an imaging target. For example, the user can generate a rendered image showing the biological tissue (a part of the biological tissue that can be shown based on the CT data) viewed from a prescribed point of view by processing or edition of volume data of the biological tissue obtained by the CT scanner. Furthermore, the user can generate, by varying the prescribed point of view in multiple directions, a plurality of rendered images showing the biological tissue viewed from multiple directions. Similarly to the case of the IOS data, the rendered image may be a two-dimensional image or a three-dimensional image.

The three-dimensional data including position information of each point in a point group indicating the surface of the biological tissue, the position information being obtained by the three-dimensional scanner, is also referred to as "IOS data," and the rendered image generated based on the IOS data is also referred to as an "IOS image" below. The three-dimensional volume data obtained by the CT scanner is also referred to as "CT data" and the rendered image generated based on the CT data is also referred to as a "CT image." The IOS image can show in a very detailed manner, a surface geometry of the biological tissue to be scanned, whereas it cannot show an internal construction (an alveolar bone, a root apex portion, or the like) that does not appear at the surface of the biological tissue. The CT image can show the hard tissue portion (the bone, the tooth, or the like) of an imaging target relatively in detail, whereas it cannot show a soft tissue portion (the skin, the gingiva, or the like) in a manner as detailed as the hard tissue portion.

The user can generate oral cavity image data by combining the IOS data and the CT data obtained for the same subject. The IOS data and the CT data are different from each other in data format. Therefore, the user generates the oral cavity image data which is combination of the IOS data and the CT data, for example, by converting the data format of the IOS data into the data format of the CT data and subjecting the three-dimensional geometry of the surface of the biological tissue to pattern matching with the use of converted data of both of them. Alternatively, the user may generate the oral cavity image data by converting the data format of the CT data into the data format of the IOS data and subjecting the three-dimensional geometry of the surface of the biological tissue to pattern matching with the use of converted data of both of them. Alternatively, the user may generate the oral cavity image data by converting the data formats of the CT data and the IOS data into a common data format and subjecting the three-dimensional geometry of the surface of the biological tissue to pattern matching with the use of converted data of both of them. How to process a data format may be determined on a program side.

The user can generate a rendered image (for example, an oral cavity image shown in Fig. 1) showing a two-dimensional biological tissue (a part of the biological tissue that can be shown based on both of the IOS data and the CT data) viewed from a prescribed point of view by processing or edition of the oral cavity image data. As shown in Fig. 1, the oral cavity image can three-dimensionally show the surface geometry of the biological tissue based on the IOS data and a tomographic structure or an appearance in the hard tissue portion (the bone, the tooth, or the like) based on the CT data. In generation of the oral cavity image, the user may adjust a luminance, a contrast, a transmittance, and the like as necessary in each of the IOS data and the CT data.

Data generation apparatus 1 may obtain the IOS data from the three-dimensional scanner, obtain the CT data from the CT scanner, and generate the oral cavity image data based on the obtained IOS data and CT data in accordance with a user input. Alternatively, data generation apparatus 1 may obtain from another apparatus, the oral cavity image data generated by the user with another apparatus, without obtaining the IOS data and the CT data.

The oral cavity image shows based on the CT data, the three-dimensional geometry of the hard tissue portion such as the alveolar bone and the root apex portion and shows based on the IOS data, the three-dimensional geometry of the soft tissue portion such as the gingiva that cannot be shown based on the CT data. The oral cavity image can thus show in detail also the soft tissue portion such as the gingiva that cannot be shown only based on the CT data, owing to supplement by the IOS data, together with the hard tissue portion such as the alveolar bone and the root apex portion.

As shown in Fig. 1, data generation apparatus 1 shows on a display 20, an oral cavity image showing the inside of the oral cavity including teeth and the gingiva. Data generation apparatus 1 can show on display 20, the oral cavity image showing the oral cavity viewed from various points of view by performing processing on the oral cavity image shown on display 20, based on a user input. Furthermore, data generation apparatus 1 can calculate a crown root ratio of a tooth designated by the user in the oral cavity image shown on display 20 and show the calculated crown root ratio. The user thus does not have to measure a depth of a periodontal pocket or measure the crown root ratio by examination of a radiographic image, and can easily and accurately measure the crown root ratio of a desired tooth.

Data generation apparatus 1 may perform segmentation for each anatomical element shown in the oral cavity image, in generating the oral cavity image based on the IOS data and the CT data. For example, segmentation may be various, such as segmentation of each of a hard tissue and a soft tissue, segmentation of each of a maxillary hard tissue and a mandibular hard tissue, segmentation of each of the dentition and the alveolar bone, segmentation of each of the dentition in the maxilla and the dentition in the mandible, and segmentation of each of teeth. For example, data generation apparatus 1 may recognize an area of the dentition in the maxilla, the dentition in the mandible, each tooth included in the dentition in the maxilla, and each tooth included in the dentition in the mandible which are segmented by the user, segment each part of them, and show the part in the oral cavity image, based on the user input.

Alternatively, data generation apparatus 1 may automatically segment each part based on artificial intelligence (AI) technology, without following the user input. For example, data generation apparatus 1 may be provided with an estimation model including a neural network for identification of the dentition in the maxilla, the dentition in the mandible, and each of teeth shown in the oral cavity image, based on the oral cavity image data. The estimation model is trained in machine learning so as to segment the dentition in the maxilla, the dentition in the mandible, and each of teeth shown in the oral cavity image based on the inputted oral cavity image data, with training data in which a plurality of oral cavity images and ground truth data indicating an area of each segmented part shown in each of the plurality of oral cavity images are defined as a set. With such an estimation model, the user can obtain the oral cavity image showing the dentition in the maxilla, the dentition in the mandible, and each of teeth segmented by the estimation model of a control device 10, by inputting the oral cavity image data to data generation apparatus 1.

### [Configuration of Data Generation Apparatus]

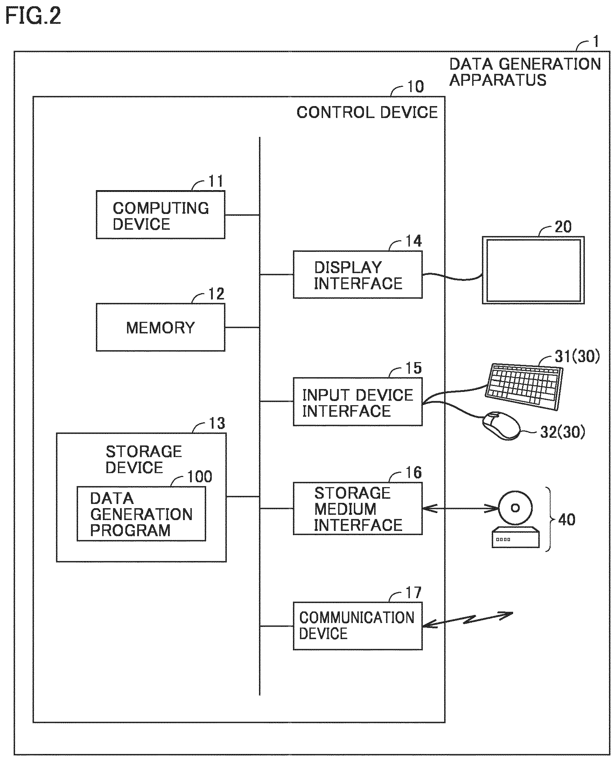

A configuration of data generation apparatus will be described with reference to Fig. 2. Fig. 2 is a block diagram showing a configuration of data generation apparatus 1. Data generation apparatus 1 may be implemented, for example, by a general-purpose computer or a dedicated computer.

As shown in Fig. 2, data generation apparatus 1 includes control device 10, display 20, and an input device 30.

Control device 10 includes a computing device 11, a memory 12, a storage device 13, a display interface 14, an input device interface 15, a storage medium interface 16, and a communication device 17.

Computing device 11 is a computing entity (computer) that performs various types of processing by executing various programs. Computing device 11 is implemented by a processor such as a central processing unit (CPU), a micro processing unit (MPU), a tensor processing unit (TPU), or a graphics processing unit (GPU). Though the processor which represents an exemplary computing device 11 performs functions to perform various types of processing by executing a program, some or all of these functions may be performed by dedicated hardware circuitry such as an application specific integrated circuit (ASIC) or a field programmable gate array (FPGA). The "processor" is not limited to a processor in a narrow sense that performs processing in accordance with a stored program architecture like the CPU, the MPU, the TPU, or the GPU, but may encompass hard-wired circuitry such as the ASIC or the FPGA. Computing device 11 is not limited to a von Neumann computer such as the CPU or the GPU but may be implemented by a non von Neumann computer such as a quantum computer or an optical computer. Computing device 11 as described above can also be read as processing circuitry that performs prescribed processing. Computing device 11 may be implemented by a single chip or a plurality of chips. Furthermore, the processor and relating processing circuitry may be implemented by a plurality of computers connected to one another through wires or wirelessly over a local area network or a wireless network. The processor and the relating processing circuitry may be implemented by a cloud computer that performs remote computation based on input data and outputs a result of computation to another device located at a remote position. Execution of various programs by computing device 11 also corresponds to various programs causing computing device 11 to perform various types of processing.

Memory 12 includes a volatile storage area (for example, a working area) where a program code or a work memory is temporarily stored in execution of various programs by computing device 11. Examples of memory 12 include a volatile memory such as a dynamic random access memory (DRAM) or a static random access memory (SRAM) or a non-volatile memory such as a read only memory (ROM) or a flash memory.

Various programs to be executed by computing device 11 or various types of data are stored in storage device 13. Storage device 13 may be implemented by one or more non-transitory computer readable media or one or more computer readable storage media. Examples of storage device 13 include a hard disk drive (HDD) and a solid state drive (SSD).

A data generation program 100 is stored in storage device 13. Contents of data generation processing for generation of oral cavity image data for calculation of the crown root ratio of a tooth and representation of the calculated crown root ratio by computing device 11 are described in data generation program 100.

Display interface 14 is an interface for connection of display 20. Display interface 14 implements input and output of data between control device 10 and display 20. For example, control device 10 causes display 20 to show the oral cavity image based on oral cavity image data through display interface 14.

Input device interface 15 is an interface for connection of input device 30 such as a keyboard 31 and a mouse 32. Input device interface 15 implements input and output of data between control device 10 and input device 30. For example, the user can use input device 30 such as keyboard 31 and mouse 32 to input a desired command through input device interface 15, so as to have control device 10 generate and edit the oral cavity image data based on the command. Inputting by the user to control device 10 through input device 30 or data (command) inputted to control device 10 by the user is also referred to as the "user input."

Storage medium interface 16 reads various types of data stored in a storage medium 40 such as a removable disc or writes various types of data into storage medium 40. For example, data generation apparatus 1 may obtain data generation program 100 from storage medium 40 through storage medium interface 16 or write oral cavity image data indicating the crown root ratio of the tooth into storage medium 40 through storage medium interface 16. Storage medium 40 may be implemented by one or more non-transitory computer readable media or one or more computer readable storage media. Control device 10 may obtain the IOS data, the CT data, or combined data of the IOS data and the CT data from storage medium 40 through storage medium interface 16.

Communication device 17 transmits and receives data to and from an external apparatus through wired or wireless communication. For example, control device 10 may receive data generation program 100 from an external apparatus through communication device 17 or transmit oral cavity image data indicating the crown root ratio of the tooth to an external apparatus through communication device 17. Control device 10 may obtain the IOS data, the CT data, or combined data of the IOS data and the CT data from an external apparatus through communication device 17.

### [Exemplary Measurement of Crown Root Ratio]

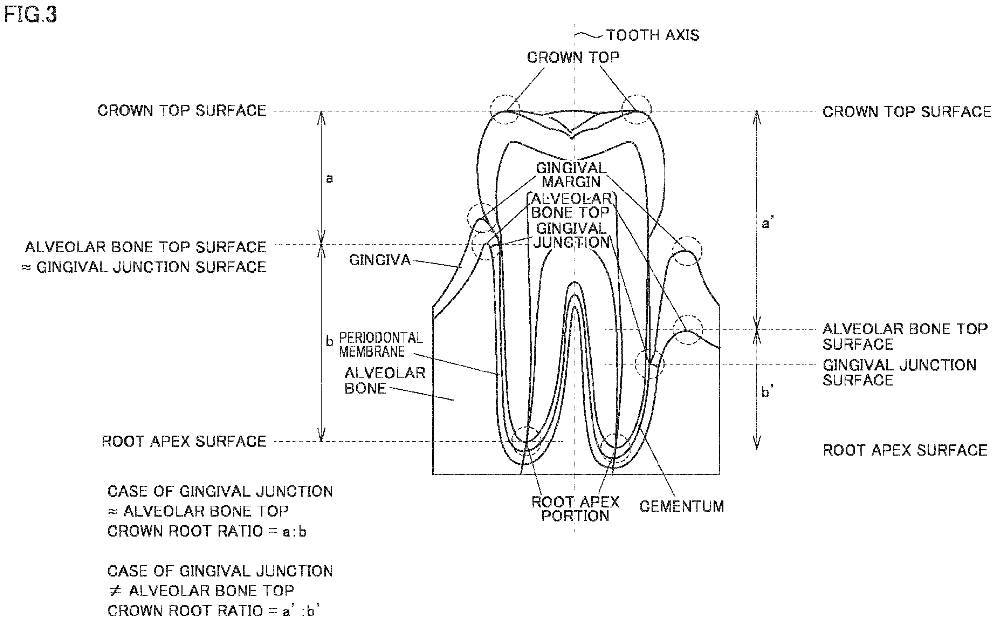

Exemplary measurement of the crown root ratio by data generation apparatus 1 will be described with reference to Figs. 3 to 7. Fig. 3 is a diagram for illustrating setting of a measurement direction and each plane for measurement of the crown root ratio. Processing by data generation apparatus 1 which will be described below is performed by execution of data generation program 100 by control device 10 (computing device 11) of data generation apparatus 1.

Fig. 3 shows a vertical cross-section of the biological tissue including the tooth and the gingiva. As shown in Fig. 3, data generation apparatus 1 sets a measurement direction in accordance with a prescribed reference for each tooth in the oral cavity image showing the biological tissue including the tooth and the gingiva. For example, the user uses input device 30 to designate for each tooth in data generation apparatus 1, a tooth axis indicating an inclination of the tooth as the prescribed reference while viewing the oral cavity image. Data generation apparatus 1 sets the measurement direction along a direction of the tooth axis designated by the user, based on the user input. The user may designate a direction other than the direction of the tooth axis as the prescribed reference in data generation apparatus 1. In this case, data generation apparatus 1 sets the measurement direction along a direction other than the direction of the tooth axis designated by the user.

When data generation apparatus 1 sets the measurement direction, it sets a plurality of planes at heights different from one another along the measurement direction, based on the user input. For example, the user uses input device 30 to designate the plurality of planes along the measurement direction in data generation apparatus 1 for each tooth while viewing the oral cavity image. Data generation apparatus 1 sets the plurality of planes designated by the user, based on the user input. The plurality of planes described above include a crown top surface, an alveolar bone top surface, a gingival junction surface, and a root apex surface that are orthogonal to the tooth axis. An image obtained by segmentation of an individual tooth may be used as an image of the tooth shown in the oral cavity image.

The crown top surface is an exemplary "first plane" and passes through a position (for example, the height) of the top of the crown portion in the measurement direction. Data generation apparatus 1 can set a spot (an exemplary "first spot") indicating the top of the crown portion on the first plane based on the user input. For example, while the user views the oral cavity image, the user uses input device 30 to designate the crown top surface in data generation apparatus 1 such that it passes through the top of the crown portion of each tooth. Data generation apparatus 1 can set the spot indicating the top of the crown portion by setting the crown top surface designated by the user in each tooth based on the user input. Data generation apparatus 1 may directly set the spot indicating the top of the crown portion based on the user input, rather than setting the crown top surface that passes through the top of the crown portion. In other words, the user may directly designate the spot indicating the top of the crown portion in data generation apparatus 1, rather than designating the crown top surface in data generation apparatus 1.

The alveolar bone top surface is an exemplary "second plane" and passes through a position (for example, the height) of the top of the alveolar bone in the measurement direction. Data generation apparatus 1 can set a spot (an exemplary "second spot") indicating the top of the alveolar bone on the second plane based on the user input. For example, while the user views the oral cavity image, the user uses input device 30 to designate the alveolar bone top surface in data generation apparatus 1 such that it passes through the top of the alveolar bone of each tooth. Data generation apparatus 1 can set the spot indicating the top of the alveolar bone by setting the alveolar bone top surface designated by the user in each tooth based on the user input. Data generation apparatus 1 may directly set the spot indicating the top of the alveolar bone based on the user input, rather than setting the alveolar bone top surface that passes through the top of the alveolar bone. In other words, the user may directly designate the spot indicating the top of the alveolar bone in data generation apparatus 1, rather than designating the alveolar bone top surface in data generation apparatus 1.

The gingival junction surface is an exemplary "second plane" and passes through a position (for example, the height) of the junction (a bottom portion of the periodontal pocket) between the tooth and the gingiva in the measurement direction. Data generation apparatus 1 can set a spot (an exemplary "second spot") indicating the junction between the tooth and the gingiva on the second plane based on the user input. For example, while the user views the oral cavity image, the user uses input device 30 to designate the gingival junction surface in data generation apparatus 1 such that it passes through the junction between the tooth and the gingiva in each tooth. Data generation apparatus 1 can set the spot indicating the junction between the tooth and the gingiva by setting the gingival junction surface designated by the user in each tooth based on the user input. Data generation apparatus 1 may directly set the spot indicating the junction between the tooth and the gingiva based on the user input, rather than setting the gingival junction surface that passes through the junction between the tooth and the gingiva. In other words, the user may directly designate the spot indicating the junction between the tooth and the gingiva in data generation apparatus 1, rather than designating the gingival junction surface in data generation apparatus 1.

The root apex surface is an exemplary "third plane" and passes through a position (for example, the height) of the root apex portion in the measurement direction. Data generation apparatus 1 can set a spot (an exemplary "third spot") indicating the root apex portion on the third plane based on the user input. For example, while the user views the oral cavity image, the user uses input device 30 to designate the root apex surface in data generation apparatus 1 such that it passes through the root apex portion of each tooth. Data generation apparatus 1 can set the spot indicating the root apex portion by setting the root apex surface designated by the user in each tooth based on the user input. Data generation apparatus 1 may directly set the spot indicating the root apex portion based on the user input, rather than setting the root apex surface that passes through the root apex portion. In other words, the user may directly designate the spot indicating the root apex portion in data generation apparatus 1, rather than designating the root apex surface in data generation apparatus 1.

When there is no periodontal pocket or the depth of the periodontal pocket is extremely small, the alveolar bone top surface and the gingival junction surface are flush or substantially flush with each other, and a difference between the position of the alveolar bone top surface and the position of the gingival junction surface is smaller than a prescribed threshold value.

Data generation apparatus 1 calculates a distance a along the measurement direction between the crown top surface and the gingival junction surface or the alveolar bone top surface as a crown length, and calculates a distance b along the measurement direction between the gingival junction surface or the alveolar bone top surface and the root apex surface as a root length. The crown length (distance a) is an exemplary "first distance." The root length (distance b) is an exemplary "second distance." Furthermore, data generation apparatus 1 calculates a ratio (a:b) between the crown length (distance a) and the root length (distance b) as the crown root ratio.

Calculation of the ratio (a:b) between the crown length (distance a) and the root length (distance b) is suitable when the alveolar bone top surface and the gingival junction surface are expected to be flush or substantially flush. When the alveolar bone top surface and the gingival junction surface are expected to be different, however, data generation apparatus 1 calculates a distance a' along the measurement direction between the crown top surface and the alveolar bone top surface as the crown length and calculates a distance b' along the measurement direction between the alveolar bone top surface and the root apex surface as the root length. Furthermore, data generation apparatus 1 calculates a ratio (a':b') between the crown length (distance a') and the root length (distance b') as the crown root ratio. For example, data generation apparatus 1 may determine whether or not the alveolar bone top surface and the gingival junction surface are flush or substantially flush by conducting pocket measurement.

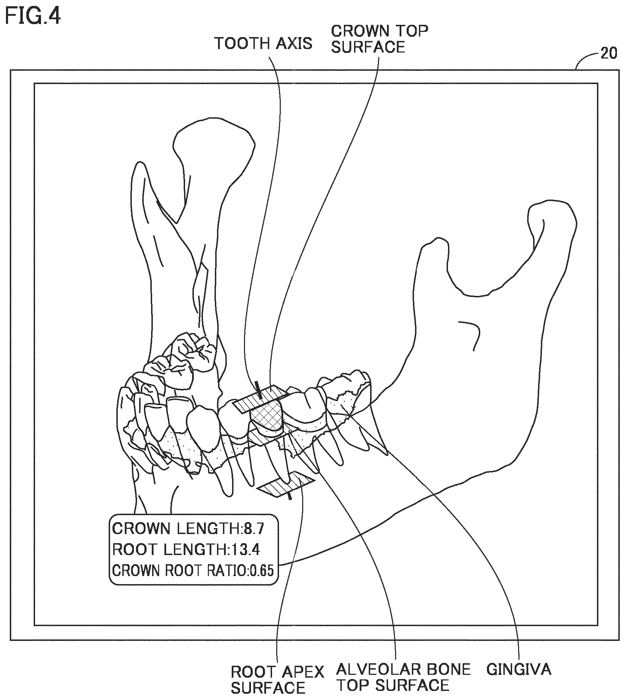

Figs. 4 to 7 are each a diagram for illustrating exemplary setting of the crown root ratio. As shown in Figs. 4 to 7, data generation apparatus 1 can show on display 20, the oral cavity image of an imaging target viewed from various points of view or the oral cavity image obtained by zoom-up or -down of the imaging target, based on the user input.

For example, as shown in Fig. 4, data generation apparatus 1 can show on display 20, the oral cavity image of the biological tissue including the tooth and the gingiva viewed from a front side of the mandible. While the user views the oral cavity image shown in Fig. 4, the user uses input device 30 to designate the tooth axis in data generation apparatus 1 for the tooth the crown root ratio of which is to be measured and to designate the crown top surface, the alveolar bone top surface, and the root apex surface in data generation apparatus 1 for that measurement target tooth. At this time, the user can use input device 30 to drag and move the tooth axis to any position to adjust the position of the tooth axis, or to drag and move each of the crown top surface, the alveolar bone top surface, and the root apex surface in the measurement direction (a tooth axis direction) to adjust the position (for example, the height) of each of the crown top surface, the alveolar bone top surface, and the root apex surface. The user can thus adjust the spot indicating the top of the crown portion, the spot indicating the top of the alveolar bone, and the spot indicating the root apex portion by adjusting the position (for example, the height) of each of the crown top surface, the alveolar bone top surface, and the root apex surface. Data generation apparatus 1 sets the tooth axis designated by the user and sets the crown top surface, the alveolar bone top surface, and the root apex surface for the measurement target tooth, based on the user input.

When data generation apparatus 1 sets the tooth axis, the crown top surface, the alveolar bone top surface, and the root apex surface based on the user input, it calculates the distance along the tooth axis between the crown top surface and the alveolar bone top surface as the crown length and calculates the distance along the tooth axis between the alveolar bone top surface and the root apex surface as the root length. In the example in Fig. 4, data generation apparatus 1 calculates the crown length of 8.7 mm and calculates the root length of 13.4 mm. Furthermore, data generation apparatus 1 calculates the ratio between the crown length and the root length as the crown root ratio and provides pop-up representation of a numerical value on display 20. In the example in Fig. 4, data generation apparatus 1 calculates the crown root ratio (crown length/root length) of 0.65 and provides pop-up representation of the numerical value on display 20. When the measurement target tooth is designated by the user input, data generation apparatus 1 adds a color in accordance with the crown root ratio to the measurement target tooth.

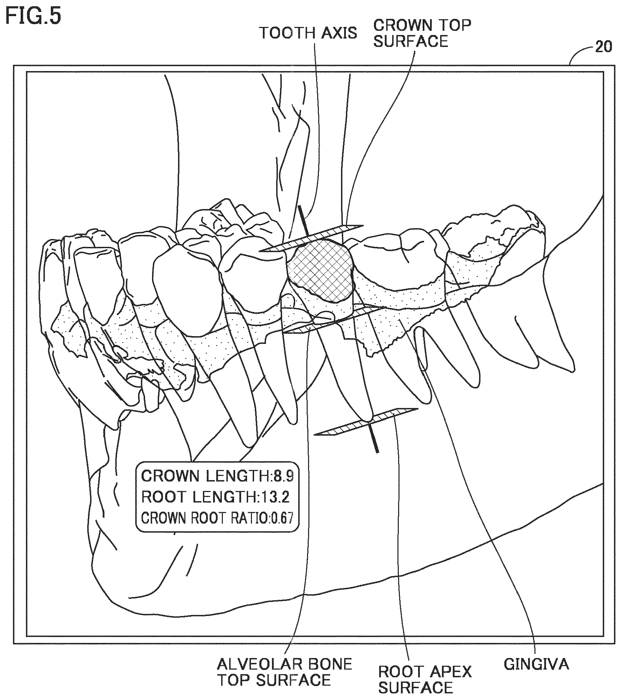

As shown in Fig. 5, data generation apparatus 1 can show on display 20, an oral cavity image obtained by zoom-up of the biological tissue including the tooth and the gingiva. For example, the user can use input device 30 to change the positions of the first spot, the second spot, and the third spot in the measurement target tooth while viewing the oral cavity image shown in Fig. 5. For example, data generation apparatus 1 can drag and move each of the crown top surface, the alveolar bone top surface, and the root apex surface to adjust the crown top surface, the alveolar bone top surface, and the root apex surface to more appropriate positions. Data generation apparatus 1 may make movement described above in the measurement direction. In particular, the biological tissue is zoomed up in the oral cavity image shown in Fig. 5 as compared with that in the oral cavity image shown in Fig. 4. Therefore, the user can set the alveolar bone top surface at an appropriate position even in a portion difficult to detect, such as the top of the alveolar bone. Data generation apparatus 1 (computing device 11) calculates the ratio between the first distance and the second distance that changes depending on positions of the first spot, the second spot, and the third spot in a calculation step which will be described later. Data generation apparatus 1 (computing device 11) calculates the ratio between the first distance and the second distance that changes depending on positions of the first plane, the second plane, and the third plane in the calculation step which will be described later.

In the example in Fig. 5, the user finely adjusts the alveolar bone top surface, so that data generation apparatus 1 calculates 8.9 mm as the crown length and calculates 13.2 mm as the root length. Data generation apparatus 1 thus calculates 0.67 as the crown root ratio and provides pop-up representation of the numerical value on display 20.

Data generation apparatus 1 can thus show on display 20, the biological tissue shown in the oral cavity image, as being zoomed-up. Therefore, the user can set the crown top surface, the alveolar bone top surface, and the root apex surface at the more appropriate positions while viewing the zoomed-up biological tissue shown in the oral cavity image. The user can thus more accurately measure the crown root ratio.

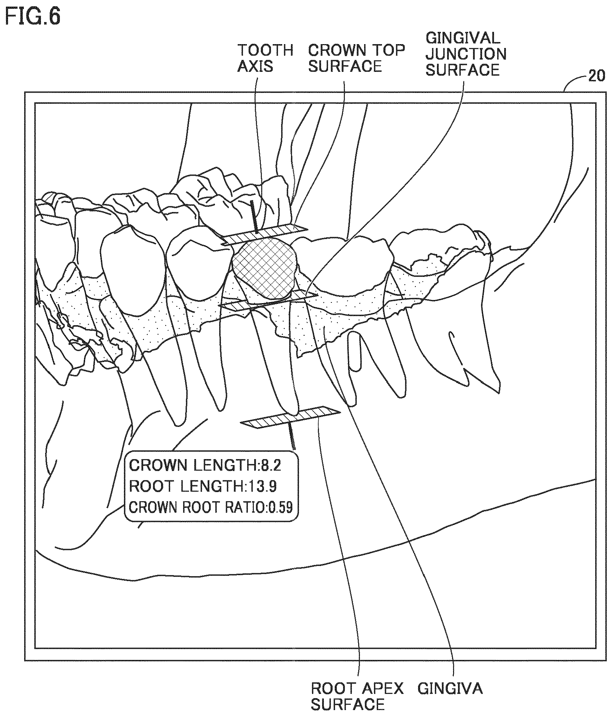

As shown in Fig. 6, the user can use input device 30 to also designate the gingival junction surface instead of the alveolar bone top surface in data generation apparatus 1. At this time, the user can use input device 30 to drag and move the gingival junction surface in the measurement direction (tooth axis direction) to adjust the position (for example, the height) of the gingival junction surface. The user can thus adjust the spot indicating the junction between the tooth and the gingiva by adjusting the position (for example, the height) of the gingival junction surface. Data generation apparatus 1 sets the tooth axis designated by the user and sets the crown top surface, the gingival junction surface, and the root apex surface for the measurement target tooth, based on the user input.

When data generation apparatus 1 sets the tooth axis, the crown top surface, the gingival junction surface, and the root apex surface based on the user input, it calculates the distance along the tooth axis between the crown top surface and the gingival junction surface as the crown length and calculates the distance along the tooth axis between the gingival junction surface and the root apex surface as the root length. In the example in Fig. 6, data generation apparatus 1 calculates 8.2 mm as the crown length and calculates 13.9 mm as the root length. Furthermore, data generation apparatus 1 calculates the ratio between the crown length and the root length as the crown root ratio and provides pop-up representation of the numerical value on display 20. In the example in Fig. 6, data generation apparatus 1 calculates 0.59 as the crown root ratio (crown length/root length) and provides pop-up representation of the numerical value on display 20.

Since data generation apparatus 1 can thus set any of the alveolar bone top surface and the gingival junction surface, the user can measure the crown root ratio with the alveolar bone top surface being defined as a boundary or measure the crown root ratio with the gingival junction surface being defined as the boundary, depending on the state of the tooth and the gingiva.

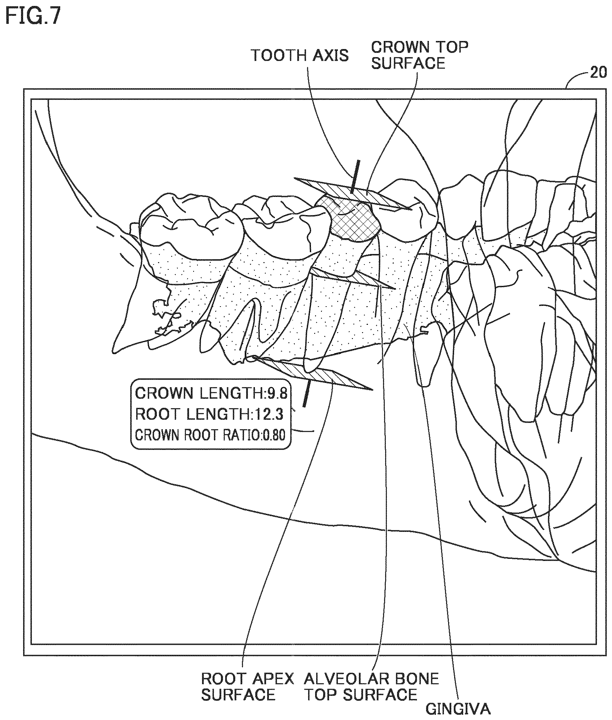

As shown in Fig. 7, data generation apparatus 1 can show on display 20, the oral cavity image in which the biological tissue including the tooth and the gingiva is seen from a rear side of the mandible. In other words, the user can calculate the crown root ratio while viewing the biological tissue from any direction, such as the rear side, without being limited to the biological tissue viewed from the front side. For example, while the user views the oral cavity image shown in Fig. 7, the user can use input device 30 to drag and move each of the crown top surface, the alveolar bone top surface, and the root apex surface of the measurement target tooth to adjust the crown top surface, the alveolar bone top surface, and the root apex surface to appropriate positions.

When data generation apparatus 1 sets the tooth axis, the crown top surface, the alveolar bone top surface, and the root apex surface based on the user input, it calculates the distance along the tooth axis between the crown top surface and the alveolar bone top surface as the crown length and calculates the distance along the tooth axis between the alveolar bone top surface and the root apex surface as the root length. In the example in Fig. 7, data generation apparatus 1 calculates 9.8 mm as the crown length and calculates 12.3 mm as the root length. Furthermore, data generation apparatus 1 calculates the ratio between the crown length and the root length as the crown root ratio and provides pop-up representation of the numerical value on display 20. In the example in Fig. 7, data generation apparatus 1 calculates 0.80 as the crown root ratio and provides pop-up representation of the numerical value on display 20.

Data generation apparatus 1 can thus show on display 20, the oral cavity image viewed from a plurality of points of view based on the user input and set the crown top surface, the alveolar bone top surface, the gingival junction surface, and the root apex surface in the oral cavity image viewed from a specific point of view selected from among the plurality of points of view, and show on display 20, the oral cavity image viewed from the plurality of points of view based on the user input and set the first spot, the second spot, and the third spot in the oral cavity image viewed from the specific point of view selected from among the plurality of points of view. Therefore, the user can measure the crown root ratio when the tooth is viewed from any direction.

### [Processing by Data Generation Apparatus]

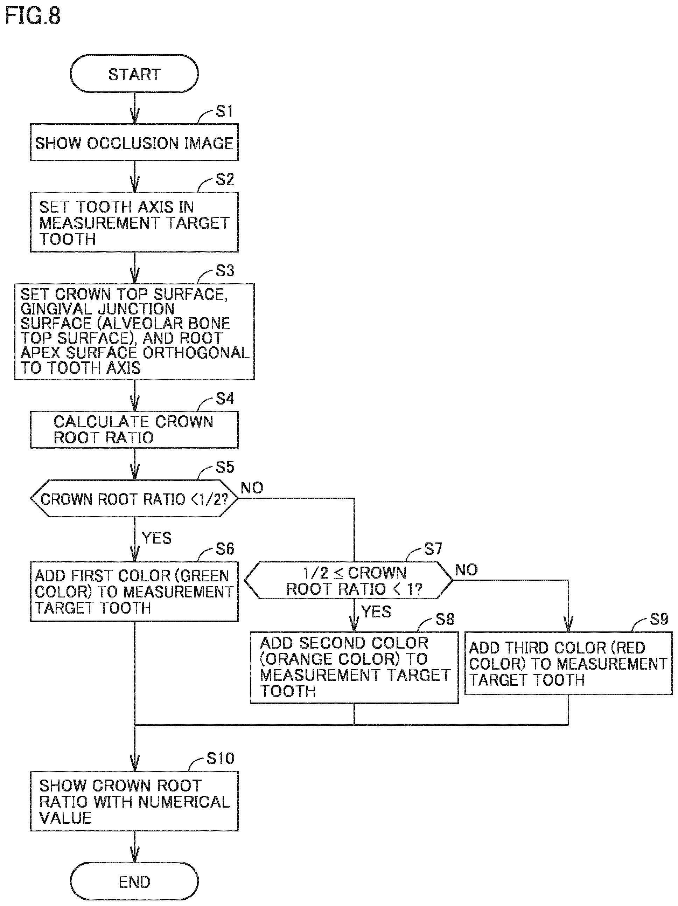

Processing performed by data generation apparatus 1 will be described with reference to Fig. 8. Fig. 8 is a flowchart for illustrating exemplary data generation processing performed by data generation apparatus 1. Each STEP (which is denoted as "S" below) shown in Fig. 8 is performed by execution of data generation program 100 by control device 10 (computing device 11) of data generation apparatus 1.

As shown in Fig. 8, data generation apparatus 1 shows on display 20, the oral cavity image three-dimensionally showing the biological tissue including the tooth and the gingiva (S1). For example, data generation apparatus 1 shows on display 20, the oral cavity image showing the biological tissue including two-dimensional tooth and gingiva viewed from a specific point of view as illustrated in Figs. 4 to 7. Processing in S1 is an exemplary "display step."

Data generation apparatus 1 sets the tooth axis in the tooth the crown root ratio of which is to be measured, in the oral cavity image shown on display 20, based on the user input provided through input device 30 (S2). Data generation apparatus 1 sets the crown top surface, the gingival junction surface or the alveolar bone top surface, and the root apex surface that are orthogonal to the tooth axis in the oral cavity image, based on the user input provided through input device 30 (S3). Processing in S3 is an exemplary "spot setting step." Data generation apparatus 1 performs processing for adding a color in accordance with the ratio between the first distance and the second distance to the measurement target tooth shown in the oral cavity image. Data generation apparatus 1 performs processing for adding a numerical value in accordance with the ratio between the first distance and the second distance to the measurement target tooth shown in the oral cavity image.

Data generation apparatus 1 calculates the crown root ratio based on the crown top surface, the gingival junction surface or the alveolar bone top surface, and the root apex surface (S4). Processing in S4 is an exemplary "calculation step." Data generation apparatus 1 determines whether or not the crown root ratio is lower than 1/2 (S5). When the crown root ratio is lower than 1/2 (YES in S5), data generation apparatus 1 adds a first color (for example, a green color) in accordance with the crown root ratio to the measurement target tooth shown in the oral cavity image (S6). In other words, in order to show that the crown root ratio has a normal value, data generation apparatus 1 shows the measurement target tooth as being colored with the first color (for example, the green color).

When the crown root ratio is equal to or higher than 1/2 (NO in S5), data generation apparatus 1 determines whether or not the crown root ratio is equal to or higher than 1/2 and lower than 1 (S7). When the crown root ratio is equal to or higher than 1/2 and lower than 1 (YES in S7), data generation apparatus 1 adds a second color (for example, an orange color) in accordance with the crown root ratio to the measurement target tooth shown in the oral cavity image (S8). In other words, in order to call attention because of the crown root ratio being closer to an abnormal value, data generation apparatus 1 shows the measurement target tooth as being colored with the second color (for example, the orange color).

When the crown root ratio is equal to or higher than 1 (NO in S7), data generation apparatus 1 adds a third color (for example, a red color) in accordance with the crown root ratio to the measurement target tooth shown in the oral cavity image (S9). In other words, in order to show that the crown root ratio has the abnormal value, data generation apparatus 1 shows the measurement target tooth as being colored with the third color (for example, the red color).

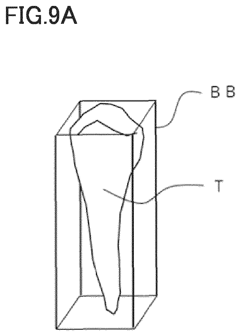

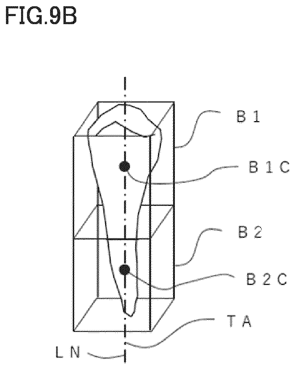

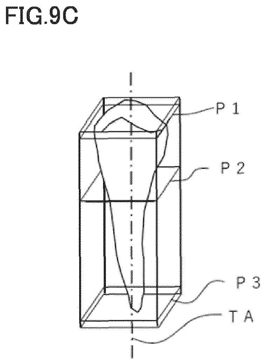

After processing in S6, S8, or S9, as illustrated in Figs. 4 to 7, data generation apparatus 1 shows on display 20, the measurement target tooth in such a manner that the numerical value in accordance with the crown root ratio is added thereto (S10). Data generation apparatus 1 may output the numerical value in accordance with the crown root ratio in a comma separated values (CSV) file or a text file. Thereafter, data generation apparatus 1 quits the present process. The root apex side of a tooth axis may be regarded as a lower side of the tooth axis, and the crown portion side of the tooth axis may be regarded as a higher side of the tooth axis. The first plane, the second plane, and the third plane are set as planes intersecting the tooth axis, at different heights, without contact with each other, and parallel to each other. The first spot, the second spot, and the third spot are located on a straight line preferably. Now this straight line on which the spots are located is called as "spots line". The spots line may be parallel to the tooth axis, and also may be non-parallel to the tooth axis. The spots line may be perpendicular to the first plane, the second plane, and the third plane, and also may intersect with the first plane, the second plane, and the third plane at an angle. It is possible to calculate the position of each of the first spot, the second spot, the third spot to be at a point of each of the first plane, the second plane, the third plane intersecting the points line. (Fig.9 can be referred as explained later). Because the ratio of distance between the spots is calculated in above-mentioned geometric conditions, the same ratio is to be calculated. Not necessarily to target spots, the ratio of distance between the planes can be calculated. Such the first spot, the second spot, and the third spot or the first plane, the second plane, and the third plane are regarded as indexes to calculate the ratio and may be called as "ratio conservation index".

As set forth above, data generation apparatus 1 can show on display 20, the oral cavity image viewed from the specific point of view selected from among the plurality of points of view and can set the crown top surface, the alveolar bone top surface or the gingival junction surface, and the root apex surface in the oral cavity image, based on the user input. Furthermore, data generation apparatus 1 can calculate the crown root ratio of the measurement target tooth based on the set crown top surface, alveolar bone top surface or gingival junction surface, and root apex surface, add the color in accordance with the calculated crown root ratio to the measurement target tooth, and show on display 20, the numerical value indicating the crown root ratio. The user thus does not have to measure the crown root ratio by calculating a value obtained by measuring the depth of the periodontal pocket or by manually designating a spot in a target shown in a radiographic image and conducting manual measurement, and can easily and accurately measure the crown root ratio of a desired tooth.

### <Modification>

The present disclosure is not limited to examples above, but can variously be modified and applied. Modifications applicable to the present disclosure will be described below.

Though data generation apparatus 1 is configured to set the tooth axis manually designated by the user, it may automatically set the tooth axis based on the AI technology or the like, without following the user input. For example, data generation apparatus 1 (control device 10) may be provided with a not-shown estimation model for estimation based on the oral cavity image, of the tooth axis of the tooth shown in the oral cavity image. The estimation model is trained in machine learning to estimate the tooth axis of the tooth shown in the oral cavity image based on inputted oral cavity image data, with training data where a plurality of oral cavity images and ground truth data indicating the tooth axis of the tooth shown in each of the plurality of oral cavity images are defined as a set. With the use of such an estimation model, the user can automatically set in the oral cavity image, the tooth axis estimated by the estimation model of control device 10 by inputting oral cavity image data to data generation apparatus 1.

Though data generation apparatus 1 is configured to set the first spot (the crown top surface) indicating the top of the crown portion, the second spot (the alveolar bone top surface or the gingival junction surface) indicating the top of the alveolar bone or the junction between the tooth and the gingiva, and the third spot (the root apex surface) indicating the root apex portion that are manually designated by the user, it may set each spot (each plane) described above automatically based on the AI technology or the like, without following the user input. For example, data generation apparatus 1 (control device 10) may be provided with a not-shown estimation model for estimation of the first spot (the crown top surface), the second spot (the alveolar bone top surface or the gingival junction surface), and the third spot (the root apex surface) for the tooth shown in the oral cavity image, based on the oral cavity image. The estimation model is trained in machine learning to estimate the first spot (the crown top surface), the second spot (the alveolar bone top surface or the gingival junction surface), and the third spot (the root apex surface) shown in the oral cavity image, based on the inputted oral cavity image data, with training data where a plurality of oral cavity images and ground truth data indicating the first spot (the crown top surface), the second spot (the alveolar bone top surface or the gingival junction surface), and the third spot (the root apex surface) in the tooth shown in each of the plurality of oral cavity images are defined as a set. With such an estimation model, the user can automatically set in the oral cavity image, the first spot (the crown top surface), the second spot (the alveolar bone top surface or the gingival junction surface) and the third spot (the root apex surface) estimated by the estimation model of control device 10, by inputting the oral cavity image data in data generation apparatus 1.

Alternatively, the estimation model described above may estimate the first spot (crown top surface) and the third spot (root apex surface) based on a result of measurement using a bounding box. For example, the estimation model has a tooth shown in each of the plurality of oral cavity images surrounded by a three-dimensional bounding box for computation during training. The estimation model is subjected to machine learning to estimate the tooth axis, the first spot, and the third spot with a shape of the bounding box being defined as the reference. For example, the estimation model divides an area occupied by the bounding box in two areas in a predetermined direction, calculates a three-dimensional center of gravity of the tooth in each area, and estimates a straight line that passes through the centers of gravity as the tooth axis. The estimation model may set two areas at predetermined positions, without necessarily dividing the area into two areas. The estimation model may set more than two areas and determine a general center of gravity. The estimation model is subjected to machine learning to set the tooth axis and to estimate a surface at which distance from an end surface on a crown side of the bounding box is the crown top surface and a surface at which distance from an end surface on a root side thereof is the root apex surface. Since image data of the alveolar bone top surface and the gingival junction surface is available for the second spot, the estimation model can estimate the position thereof by using this data.

Figs. 9A, 9B, 9C are diagrams showing exemplary use of the bounding box. As shown in Fig. 9A, the estimation model has image data of a tooth T surrounded by a bounding box BB. The estimation model has tooth T surrounded such that tooth T is in contact with all surfaces of bounding box BB and tooth T leans least. As shown in Fig. 9B, the estimation model divides bounding box BB into two areas which are an area B1 and an area B2. The estimation model determines a three-dimensional center of gravity B1C of tooth T in area B1 and a three-dimensional center of gravity B2C of tooth T in area B2, and sets a line LN that passes through both of them as a tooth axis TA. As shown in Fig. 9C, the estimation model sets a first plane P1 which is located at a specific distance from a shown upper surface of bounding box BB and is orthogonal to tooth axis TA and a third plane P3 which is located at a specific distance from a shown lower surface and is orthogonal to tooth axis TA. Since there is image data of the alveolar bone top surface and the gingival junction surface available for a second plane P2, a position thereof is set by using this data. First plane P1 is a plane corresponding to the crown top surface. Second plane P2 is a plane corresponding to the alveolar bone top surface or the gingival junction surface. Third plane P3 is a plane corresponding to the root apex surface. Namely, the first spot is a spot on first plane P1 (crown top surface) passing through the top of the crown. The second spot is a spot on second plane P2 (alveolar bone top surface) passing through the top of the alveolar bone. Alternatively, the second spot is a spot on second plane (gingival junction surface) passing through the junction between a tooth and a gingiva. The third spot is a spot on third plane P3 (root apex surface) passing through the root apex. It is possible to calculate the position of each of the first spot, the second spot, the third spot to be on the line LN and to be at a point of each of the first plane, the second plane, the third plane intersecting the line LN. Thus, the estimation model performs machine learning in such a manner that first plane P1 (crown top surface), second plane P2 (alveolar bone top surface or gingival junction surface), and third plane P3 (root apex surface) that are defined based on the shape of the bounding box are used to estimate the first spot, the second spot, and the third spot respectively.

The configuration of data generation apparatus 1 described above may be provided in X-ray equipment (CT imager). For example, the X-ray equipment includes an imaging unit to image the maxilla and the mandible of a subject. The X-ray equipment may obtain CT data representing the hard tissue portion (the bone, the teeth, or the like) around the maxilla and the mandible of the subject by imaging by the imaging unit, generate the oral cavity image based on the obtained CT data and the IOS data obtained by a three-dimensional scanner, and show the oral cavity image on display 20. Furthermore, the X-ray equipment may set the first spot (the crown top surface), the second spot (the alveolar bone top surface or the gingival junction surface), and the third spot (the root apex surface) in the oral cavity image shown on display 20 based on the user input and calculate the crown root ratio. The crown root ratio may also be calculated by setting the first plane, the second plane, and the third plane, based on the user input.

It should be understood that the embodiment disclosed herein is illustrative and non-restrictive in every respect. The present invention is defined by the claimed subject-matter rather than the description above and is intended to include any modifications within the claimed subject-matter. The configuration illustrated in the present embodiments and the configuration illustrated in the modifications can be combined as appropriate.

## Claims

1. A data generation program (100) configured to generate image data for checking a state of a biological tissue including a tooth and a gingiva, the data generation program (100) configured to cause a computer to perform:
a display step of showing on a display (20), an oral cavity image three-dimensionally showing an oral cavity including the biological tissue;
a plane setting step of setting in the oral cavity image, a crown top surface as a first plane (P1), a gingival junction surface or an alveolar bone top surface as a second plane (P2), and a root apex surface as a third plane (P3) that are orthogonal to a tooth axis,
a spot setting step of setting in the oral cavity image, a first spot indicating a top of a crown portion, a second spot indicating a top of an alveolar bone or a junction between the tooth and the gingiva, and a third spot indicating a root apex portion, based on a user input or machine learning; wherein
the first spot is a spot on the first plane (P1) that passes through the top of the crown portion,
the second spot is a spot on the second plane (P2) that passes through the top of the alveolar bone or the junction between the tooth and the gingiva,
the third spot is a spot on the third plane (P3) that passes through the root apex portion, and
a calculation step of calculating a ratio between a first distance between the first spot and the second spot and a second distance between the second spot and the third spot.

2. The data generation program (100) according to claim 1, wherein
the calculation step includes causing the computer to move the first spot, the second spot, and the third spot in a measurement direction based on the user input and to calculate the ratio between the first distance and the second distance that changes depending on positions of the first spot, the second spot, and the third spot.

3. The data generation program (100) according to claim 2, wherein
the measurement direction is a direction along a tooth axis.

4. The data generation program (100) according to claim 3, being configured to further cause the computer
to set the tooth axis designated by a user based on the user input, or
to set the tooth axis with an estimation model for estimation of the tooth axis based on the oral cavity image.

5. The data generation program (100) according to any one of claims 2 to 4, wherein the calculation step includes causing the computer to move the first plane (P1), the second plane (P2), and the third plane (P3) in the measurement direction based on the user input and to calculate the ratio between the first distance and the second distance that changes depending on positions of the first plane (P1), the second plane (P2), and the third plane (P3).

6. The data generation program (100) according to any one of claims 1 to 5, being configured to further cause the computer
to set the first spot, the second spot, and the third spot designated by a user based on the user input.

7. The data generation program (100) according to any one of claims 1 to 5, being configured to further cause the computer
to set the first spot, the second spot, and the third spot with an estimation model for estimation of the first spot, the second spot, and the third spot based on the oral cavity image.

8. The data generation program (100) according to claim 7, wherein
the estimation model has a measurement target tooth surrounded by a bounding box (BB), and estimates the tooth axis, the first spot, and the third spot with a shape of the bounding box (BB) being defined as a reference.

9. The data generation program (100) according to any one of claims 1 to 8, wherein
the spot setting step includes causing the computer
to have the oral cavity image viewed from a plurality of points of view shown on the display (20) based on the user input, and
to set the first spot, the second spot, and the third spot in the oral cavity image viewed from a specific point of view selected from among the plurality of points of view.

10. The data generation program (100) according to any one of claims 1 to 9, being configured to further cause the computer to add a color in accordance with the ratio between the first distance and the second distance to the tooth shown in the oral cavity image.

11. The data generation program (100) according to any one of claims 1 to 10, being configured to further cause the computer to add a numerical value in accordance with the ratio between the first distance and the second distance to the tooth shown in the oral cavity image.

12. The data generation program (100) according to any one of claims 1 to 11, wherein
the oral cavity image is generated based on optical scanner data obtained by an optical scanner, the optical scanner data including position information of each point in a point group indicating a surface of the biological tissue, and computed tomography, CT, data obtained by CT scan of the biological tissue.

13. The data generation program (100) according to any one of claims 1 to 12, being configured to further cause the computer to perform segmentation for each anatomical element shown in the oral cavity image.

14. A data generation method of generating image data for checking a state of a biological tissue including a tooth and a gingiva, the data generation method comprising, as processing to be performed by a computer:
a display step of showing on a display (20), an oral cavity image three-dimensionally showing inside of an oral cavity including the biological tissue;
a plane setting step of setting in the oral cavity image, a crown top surface as a first plane (P1), a gingival junction surface or an alveolar bone top surface as a second plane (P2), and a root apex surface as a third plane (P3) that are orthogonal to a tooth axis,
a spot setting step of setting in the oral cavity image, a first spot indicating a top of a crown portion, a second spot indicating a top of an alveolar bone or a junction between the tooth and the gingiva, and a third spot indicating a root apex portion, based on a user input or machine learning; wherein
the first spot is a spot on the first plane (P1) that passes through the top of the crown portion,
the second spot is a spot on the second plane (P2) that passes through the top of the alveolar bone or the junction between the tooth and the gingiva,
the third spot is a spot on the third plane (P3) that passes through the root apex portion, and
a calculation step of calculating a ratio between a first distance between the first spot and the second spot and a second distance between the second spot and the third spot.

15. A data generation apparatus configured to generate image data for checking a state of a biological tissue including a tooth and a gingiva, the data generation apparatus comprising:
a display (20) configured to show an image;
an input device (30) configured to receive a user input; and
a control device (10) configured to control the display (20) based on the user input, wherein
the control device (10) is configured to
cause the display (20) to show an oral cavity image three-dimensionally showing inside of an oral cavity including the biological tissue,
set in the oral cavity image, a crown top surface as a first plane (P1), a gingival junction surface or an alveolar bone top surface as a second plane (P2), and a root apex surface as a third plane (P3) that are orthogonal to a tooth axis,
set in the oral cavity image, a first spot indicating a top of a crown portion, a second spot indicating a top of an alveolar bone or a junction between the tooth and the gingiva, and a third spot indicating a root apex portion, based on the user input or machine learning, wherein
the first spot is a spot on the first plane (P1) that passes through the top of the crown portion,
the second spot is a spot on the second plane (P2) that passes through the top of the alveolar bone or the junction between the tooth and the gingiva,
the third spot is a spot on the third plane (P3) that passes through the root apex portion, and
calculate a ratio between a first distance between the first spot and the second spot and a second distance between the second spot and the third spot.

## Patentansprüche

1. Datenerzeugungsprogramm (100), das konfiguriert ist, um Bilddaten zum Prüfen eines Zustands eines biologischen Gewebes, das einen Zahn und ein Zahnfleisch enthält, zu erzeugen, wobei das Datenerzeugungsprogramm (100) konfiguriert ist, um einen Computer zu veranlassen, Folgendes durchzuführen:
einen Anzeigeschritt des dreidimensionalen Zeigens eines Mundhöhlenbilds, das eine Mundhöhle zeigt, die das biologische Gewebe enthält, auf einer Anzeige (20);
einen Ebeneneinstellschritt des Einstellens einer Kronenoberseite als eine erste Ebene (P1), einer Zahnfleischverbindungsfläche oder einer Alveolarknochenoberseite als eine zweite Ebene (P2) und einer Wurzelscheitelfläche als eine dritte Ebene (P3), die orthogonal zu einer Zahnachse sind, in dem Mundhöhlenbild,
einen Punkteinstellschritt des Einstellens eines ersten Punkts, der eine Oberseite eines Kronenabschnitts angibt, eines zweiten Punkts, der eine Oberseite eines Alveolarknochens oder eine Verbindung zwischen dem Zahn und dem Zahnfleisch angibt, und eines dritten Punkts, der einen Wurzelscheitelabschnitt angibt, basierend auf einer Benutzereingabe oder maschinellem Lernen in dem Mundhöhlenbild; wobei
der erste Punkt ein Punkt auf der ersten Ebene (P1) ist, der durch die Oberseite des Kronenabschnitts verläuft,
der zweite Punkt ein Punkt auf der zweiten Ebene (P2) ist, der durch die Oberseite des Alveolarknochens oder die Verbindung zwischen dem Zahn und dem Zahnfleisch verläuft,
der dritte Punkt ein Punkt auf der dritten Ebene (P3) ist, der durch den Wurzelscheitelabschnitt verläuft, und
einen Berechnungsschritt des Berechnens eines Verhältnisses zwischen einem ersten Abstand zwischen dem ersten Punkt und dem zweiten Punkt und einem zweiten Abstand zwischen dem zweiten Punkt und dem dritten Punkt.

2. Datenerzeugungsprogramm (100) nach Anspruch 1, wobei
der Berechnungsschritt enthält, den Computer zu veranlassen, den ersten Punkt, den zweiten Punkt und den dritten Punkt basierend auf der Benutzereingabe in eine Messrichtung zu bewegen und das Verhältnis zwischen dem ersten Abstand und dem zweiten Abstand, das sich abhängig von Positionen des ersten Punkts, des zweiten Punkts und des dritten Punkts ändert, zu berechnen.

3. Datenerzeugungsprogramm (100) nach Anspruch 2, wobei
die Messrichtung eine Richtung entlang einer Zahnachse ist.

4. Datenerzeugungsprogramm (100) nach Anspruch 3, das konfiguriert ist, um ferner den Computer zu veranlassen,
die von einem Benutzer bezeichnete Zahnachse basierend auf der Benutzereingabe einzustellen, oder
die Zahnachse mit einem Schätzmodell zum Schätzen der Zahnachse basierend auf dem Mundhöhlenbild einzustellen.

5. Datenerzeugungsprogramm (100) nach einem der Ansprüche 2 bis 4, wobei der Berechnungsschritt enthält, den Computer zu veranlassen, die erste Ebene (P1), die zweite Ebene (P2) und die dritte Ebene (P3) basierend auf der Benutzereingabe in die Messrichtung zu bewegen und das Verhältnis zwischen dem ersten Abstand und dem zweiten Abstand, das sich abhängig von Positionen der ersten Ebene (P1), der zweiten Ebene (P2) und der dritten Ebene (P3) ändert, zu berechnen.

6. Datenerzeugungsprogramm (100) nach einem der Ansprüche 1 bis 5, das konfiguriert ist, um ferner den Computer zu veranlassen,
den ersten Punkt, den zweiten Punkt und den dritten Punkt, die von einem Benutzer bezeichnet werden, basierend auf der Benutzereingabe einzustellen.

7. Datenerzeugungsprogramm (100) nach einem der Ansprüche 1 bis 5, das konfiguriert ist, um ferner den Computer zu veranlassen,
den ersten Punkt, den zweiten Punkt und den dritten Punkt mit einem Schätzmodell zum Schätzen des ersten Punkts, des zweiten Punkts und des dritten Punkts basierend auf dem Mundhöhlenbild einzustellen.

8. Datenerzeugungsprogramm (100) nach Anspruch 7, wobei
das Schätzmodell einen Messzielzahn aufweist, der von einem Begrenzungskasten (BB) umgeben ist, und die Zahnachse, den ersten Punkt und den dritten Punkt schätzt, wobei eine Form des Begrenzungskastens (BB) als eine Referenz definiert ist.

9. Datenerzeugungsprogramm (100) nach einem der Ansprüche 1 bis 8, wobei
der Punkteinstellschritt enthält, den Computer zu veranlassen,
das Mundhöhlenbild basierend auf der Benutzereingabe aus einer Vielzahl von auf der Anzeige (20) gezeigten Blickpunkten betrachtet zu lassen, und
den ersten Punkt, den zweiten Punkt und den dritten Punkt in dem Mundhöhlenbild, betrachtet aus einem spezifischen Blickpunkt, der aus der Vielzahl von Blickpunkten ausgewählt ist, einzustellen.

10. Datenerzeugungsprogramm (100) nach einem der Ansprüche 1 bis 9, das konfiguriert ist, um ferner den Computer zu veranlassen, eine Farbe gemäß dem Verhältnis zwischen dem ersten Abstand und dem zweiten Abstand zu dem in dem Mundhöhlenbild gezeigten Zahn hinzuzufügen.

11. Datenerzeugungsprogramm (100) nach einem der Ansprüche 1 bis 10, das konfiguriert ist, um ferner den Computer zu veranlassen, einen numerischen Wert gemäß dem Verhältnis zwischen dem ersten Abstand und dem zweiten Abstand zu dem in dem Mundhöhlenbild gezeigten Zahn hinzuzufügen.

12. Datenerzeugungsprogramm (100) nach einem der Ansprüche 1 bis 11, wobei
das Mundhöhlenbild basierend auf optischen Scannerdaten, die durch einen optischen Scanner erhalten werden, wobei die optischen Scannerdaten Positionsinformationen jedes Punkts in einer Punktgruppe, die eine Oberfläche des biologischen Gewebes angibt, enthalten, und Computertomografie-, CT-, Daten, die durch CT-Scan des biologischen Gewebes erhalten werden, erzeugt wird.

13. Datenerzeugungsprogramm (100) nach einem der Ansprüche 1 bis 12, das konfiguriert ist, um ferner den Computer zu veranlassen, Segmentierung für jedes in dem Mundhöhlenbild gezeigte anatomische Element durchzuführen.

14. Datenerzeugungsverfahren zum Erzeugen von Bilddaten zum Prüfen eines Zustands eines biologischen Gewebes, das einen Zahn und ein Zahnfleisch enthält, wobei das Datenerzeugungsverfahren als durch einen Computer durchzuführende Verarbeitung aufweist:
einen Anzeigeschritt des dreidimensionalen Zeigens eines Mundhöhlenbilds, das das Innere einer Mundhöhle zeigt, die das biologische Gewebe enthält, auf einer Anzeige (20);
einen Ebeneneinstellschritt des Einstellens einer Kronenoberseite als eine erste Ebene (P1), einer Zahnfleischverbindungsfläche oder einer Alveolarknochenoberseite als eine zweite Ebene (P2) und einer Wurzelscheitelfläche als eine dritte Ebene (P3), die orthogonal zu einer Zahnachse sind, in dem Mundhöhlenbild,
einen Punkteinstellschritt des Einstellens eines ersten Punkts, der eine Oberseite eines Kronenabschnitts angibt, eines zweiten Punkts, der eine Oberseite eines Alveolarknochens oder eine Verbindung zwischen dem Zahn und dem Zahnfleisch angibt, und eines dritten Punkts, der einen Wurzelscheitelabschnitt angibt, basierend auf einer Benutzereingabe oder maschinellem Lernen in dem Mundhöhlenbild; wobei
der erste Punkt ein Punkt auf der ersten Ebene (P1) ist, der durch die Oberseite des Kronenabschnitts verläuft,
der zweite Punkt ein Punkt auf der zweiten Ebene (P2) ist, der durch die Oberseite des Alveolarknochens oder die Verbindung zwischen dem Zahn und dem Zahnfleisch verläuft,
der dritte Punkt ein Punkt auf der dritten Ebene (P3) ist, der durch den Wurzelscheitelabschnitt verläuft, und
einen Berechnungsschritt des Berechnens eines Verhältnisses zwischen einem ersten Abstand zwischen dem ersten Punkt und dem zweiten Punkt und einem zweiten Abstand zwischen dem zweiten Punkt und dem dritten Punkt.

15. Datenerzeugungsvorrichtung, die konfiguriert ist, um Bilddaten zum Prüfen eines Zustands eines biologischen Gewebes, das einen Zahn und ein Zahnfleisch enthält, zu erzeugen, wobei die Datenerzeugungsvorrichtung aufweist:
eine Anzeige (20), die konfiguriert ist, um ein Bild zu zeigen;
eine Eingabevorrichtung (30), die konfiguriert ist, um eine Benutzereingabe zu empfangen; und
eine Steuervorrichtung (10), die konfiguriert ist, um die Anzeige (20) basierend auf der Benutzereingabe zu steuern, wobei
die Steuervorrichtung (10) konfiguriert ist, um
die Anzeige (20) zu veranlassen, ein Mundhöhlenbild, das das Innere einer Mundhöhle zeigt, die das biologische Gewebe enthält, dreidimensional zu zeigen,
eine Kronenoberseite als eine erste Ebene (P1), eine Zahnfleischverbindungsfläche oder eine Alveolarknochenoberseite als eine zweite Ebene (P2) und eine Wurzelscheitelfläche als eine dritte Ebene (P3), die orthogonal zu einer Zahnachse sind, in dem Mundhöhlenbild einzustellen,
einen ersten Punkt, der eine Oberseite eines Kronenabschnitts angibt, einen zweiten Punkt, der eine Oberseite eines Alveolarknochens oder eine Verbindung zwischen dem Zahn und dem Zahnfleisch angibt, und einen dritten Punkt, der einen Wurzelscheitelabschnitt angibt, basierend auf der Benutzereingabe oder maschinellem Lernen in dem Mundhöhlenbild einzustellen, wobei
der erste Punkt ein Punkt auf der ersten Ebene (P1) ist, der durch die Oberseite des Kronenabschnitts verläuft,
der zweite Punkt ein Punkt auf der zweiten Ebene (P2) ist, der durch die Oberseite des Alveolarknochens oder die Verbindung zwischen dem Zahn und dem Zahnfleisch verläuft,
der dritte Punkt ein Punkt auf der dritten Ebene (P3) ist, der durch den Wurzelscheitelabschnitt verläuft, und
ein Verhältnis zwischen einem ersten Abstand zwischen dem ersten Punkt und dem zweiten Punkt und einem zweiten Abstand zwischen dem zweiten Punkt und dem dritten Punkt zu berechnen.

## Revendications

1. Programme de génération de données (100) configuré pour générer des données d'image pour vérifier un état d'un tissu biologique incluant une dent et une gencive, le programme de génération de données (100) étant configuré pour amener un ordinateur à effectuer :
une étape d'affichage pour présenter sur un écran (20) une image de cavité buccale montrant en trois dimensions une cavité buccale incluant le tissu biologique ;
une étape de définition de plans pour définir dans l'image de cavité buccale une surface supérieure de couronne en tant que premier plan (P1), une surface de jonction gingivale ou une surface supérieure d'os alvéolaire en tant que deuxième plan (P2) et une surface d'apex radiculaire en tant que troisième plan (P3) qui sont orthogonales à un axe de dent,
une étape de définition de points pour définir dans l'image de cavité buccale un premier point indiquant un sommet d'une partie de couronne, un deuxième point indiquant un sommet d'un os alvéolaire ou une jonction entre la dent et la gencive et un troisième point indiquant une partie d'apex radiculaire, sur la base d'une entrée utilisateur ou d'un apprentissage automatique ; dans lequel
le premier point est un point sur le premier plan (P1) qui passe par le sommet de la partie de couronne,
le deuxième point est un point sur le deuxième plan (P2) qui passe par le sommet de l'os alvéolaire ou la jonction entre la dent et la gencive,
le troisième point est un point sur le troisième plan (P3) qui passe par la partie d'apex radiculaire, et
une étape de calcul pour calculer un rapport entre une première distance entre le premier point et le deuxième point et une seconde distance entre le deuxième point et le troisième point.

2. Programme de génération de données (100) selon la revendication 1, dans lequel
l'étape de calcul inclut le fait d'amener l'ordinateur à déplacer le premier point, le deuxième point et le troisième point dans une direction de mesure sur la base de l'entrée utilisateur et à calculer le rapport entre la première distance et la seconde distance qui change en fonction des positions du premier point, du deuxième point et du troisième point.

3. Programme de génération de données (100) selon la revendication 2, dans lequel
la direction de mesure est une direction le long d'un axe de dent.

4. Programme de génération de données (100) selon la revendication 3, configuré pour amener en outre l'ordinateur
à définir l'axe de dent désigné par un utilisateur sur la base de l'entrée utilisateur, ou
à définir l'axe de dent avec un modèle d'estimation pour estimer l'axe de dent sur la base de l'image de cavité buccale.

5. Programme de génération de données (100) selon l'une des revendications 2 à 4, dans lequel
l'étape de calcul inclut le fait d'amener l'ordinateur à déplacer le premier plan (P1), le deuxième plan (P2) et le troisième plan (P3) dans la direction de mesure sur la base de l'entrée utilisateur et à calculer le rapport entre la première distance et la seconde distance qui change en fonction des positions du premier plan (P1), du deuxième plan (P2) et du troisième plan (P3).

6. Programme de génération de données (100) selon l'une des revendications 1 à 5, configuré pour amener en outre l'ordinateur
à définir le premier point, le deuxième point et le troisième point désignés par un utilisateur sur la base de l'entrée utilisateur.

7. Programme de génération de données (100) selon l'une des revendications 1 à 5, configuré pour amener en outre l'ordinateur
à définir le premier point, le deuxième point et le troisième point avec un modèle d'estimation pour estimer le premier point, le deuxième point et le troisième point sur la base de l'image de cavité buccale.

8. Programme de génération de données (100) selon la revendication 7, dans lequel
le modèle d'estimation présente une dent cible de mesure entourée d'un cadre de contour (BB), et estime l'axe de dent, le premier point et le troisième point selon une forme du cadre de contour (BB) définie comme référence.

9. Programme de génération de données (100) selon l'une des revendications 1 à 8, dans lequel l'étape de définition de point inclut le fait d'amener l'ordinateur
à permettre de visualiser l'image de cavité buccale d'une pluralité de points de vue affichés sur l'écran (20) sur la base de l'entrée utilisateur, et
à définir le premier point, le deuxième point et le troisième point dans l'image de cavité buccale observée d'un point de vue spécifique sélectionné parmi la pluralité de points de vue.

10. Programme de génération de données (100) selon l'une des revendications 1 à 9, configuré pour amener en outre l'ordinateur à ajouter une couleur conformément au rapport entre la première distance et la seconde distance à la dent représentée sur l'image de cavité buccale.

11. Programme de génération de données (100) selon l'une des revendications 1 à 10, configuré pour amener en outre l'ordinateur à ajouter une valeur numérique conformément au rapport entre la première distance et la seconde distance à la dent représentée sur l'image de cavité buccale.

12. Programme de génération de données (100) selon l'une des revendications 1 à 11, dans lequel
l'image de cavité buccale est générée sur la base de données de scanner optique obtenues par un scanner optique, les données de scanner optique incluant des informations de position de chaque point dans un groupe de points indiquant une surface du tissu biologique, et des données de tomodensitométrie, TDM, obtenues par balayage de tomodensitométrie du tissu biologique.

13. Programme de génération de données (100) selon l'une des revendications 1 à 12, configuré pour amener en outre l'ordinateur à effectuer une segmentation pour chaque élément anatomique représenté sur l'image de cavité buccale.

14. Procédé de génération de données pour générer des données d'image afin de vérifier un état d'un tissu biologique incluant une dent et une gencive, le procédé de génération de données comprenant, en tant que traitement à effectuer par un ordinateur :
une étape d'affichage pour présenter sur un écran (20) une image de cavité buccale montrant en trois dimensions l'intérieur d'une cavité buccale incluant le tissu biologique ;
une étape de définition de plans pour définir dans l'image de cavité buccale une surface supérieure de couronne en tant que premier plan (P1), une surface de jonction gingivale ou une surface supérieure d'os alvéolaire en tant que deuxième plan (P2) et une surface d'apex radiculaire en tant que troisième plan (P3) qui sont orthogonales à un axe de dent,
une étape de définition de points pour définir dans l'image de cavité buccale un premier point indiquant un sommet d'une partie de couronne, un deuxième point indiquant un sommet d'un os alvéolaire ou une jonction entre la dent et la gencive et un troisième point indiquant une partie d'apex radiculaire, sur la base d'une entrée utilisateur ou d'un apprentissage automatique ; dans lequel
le premier point est un point sur le premier plan (P1) qui passe par le sommet de la partie de couronne,
le deuxième point est un point sur le deuxième plan (P2) qui passe par le sommet de l'os alvéolaire ou la jonction entre la dent et la gencive,
le troisième point est un point sur le troisième plan (P3) qui passe par la partie d'apex radiculaire, et
une étape de calcul pour calculer un rapport entre une première distance entre le premier point et le deuxième point et une seconde distance entre le deuxième point et le troisième point.

15. Appareil de génération de données configuré pour générer des données d'image pour vérifier un état d'un tissu biologique incluant une dent et une gencive, l'appareil de génération de données comprenant :
un écran (20) configuré pour afficher une image ;
un dispositif d'entrée (30) configuré pour recevoir une entrée utilisateur ; et
un dispositif de commande (10) configuré pour commander l'écran (20) sur la base de l'entrée utilisateur, dans lequel
le dispositif de commande (10) est configuré pour
amener l'écran (20) à présenter une image de cavité buccale montrant en trois dimensions l'intérieur d'une cavité buccale incluant le tissu biologique,
définir dans l'image de cavité buccale une surface supérieure de couronne en tant que premier plan (P1), une surface de jonction gingivale ou une surface supérieure d'os alvéolaire en tant que deuxième plan (P2) et une surface d'apex radiculaire en tant que troisième plan (P3) qui sont orthogonales à un axe dentaire,
définir dans l'image de cavité buccale un premier point indiquant un sommet d'une partie de couronne, un deuxième point indiquant un sommet d'un os alvéolaire ou une jonction entre la dent et la gencive, et un troisième point indiquant une partie d'apex radiculaire, sur la base de l'entrée utilisateur ou d'un apprentissage automatique, dans lequel
le premier point est un point sur le premier plan (P1) qui passe par le sommet de la partie de couronne,
le deuxième point est un point sur le deuxième plan (P2) qui passe par le sommet de l'os alvéolaire ou la jonction entre la dent et la gencive,
le troisième point est un point sur le troisième plan (P3) qui passe par la partie d'apex radiculaire, et
calculer un rapport entre une première distance entre le premier point et le deuxième point et une seconde distance entre le deuxième point et le troisième point.
